# EUROPÄISCHE PATENTSCHRIFT

(11) **EP 0 717 048 B1**
(45) Veröffentlichungstag und Bekanntmachung des Hinweises auf die Patenterteilung: **30.09.1998**
(21) Anmeldenummer: 95119338.2
(22) Anmeldetag: 07.12.1995
(51) Int. Cl.: C07K 7/64, A61K 38/12

(54) **Cyclohexapeptide und deren Mischungen, Verfahren zur Herstellung sowie deren Verwendung**
Cyclic hexapeptides and their mixtures, process for their preparation and their use
Hexapeptides cycliques et leurs mélanges, procédé pour leur préparation et leur emploi

(30) Priorität: 13.12.1994 DE 4444260
(43) Veröffentlichungstag der Anmeldung: 19.06.1996
(73) Patentinhaber: HOECHST AKTIENGESELLSCHAFT, 65929 Frankfurt am Main (DE)
(72) Erfinder: Henke, Stephan, Dr., D-65719 Hofheim (DE); Jordan, Birgit, Dr., D-65795 Hattersheim (DE); Knolle, Jochen, Dr., D-65830 Kriftel (DE); Lauffer, Leander, Dr., D-35075 Gladenbach (DE); Feiertag, Susanne, D-72070 Tübingen (DE); Wiesmüller, Karl-Heinz, Dr., D-72070 Tübingen (DE); Jung, Günther, Prof. Dr., D-72076 Tübingen (DE)

(56) Entgegenhaltungen:
- FR-A- 2 360 562

## Beschreibung

Aus FR-A 2 360 562 sind Cyclohexapetide bekannt, die zur Verbesserung der Futterverwertung bei Wiederkäuern eingesetzt werden können.

Die Erfindung betrifft Verbindungen der Formel I cyclo(A-B-C-E-F-(D)-Ala)    (I) in welcher
A und B unabhängig voneinander gleich oder verschieden sein können und IIe, Leu, Val, Phe und Tyr bedeuten und C, E, und F unabhängig voneinander gleich oder verschieden sein können und den Rest einer natürlichen Aminosäure außer Cystein und Tryptophan entsprechen, sowie deren physiologisch Verträglichen Salze.

Die Erfindung betrifft ferner Verbindungen der Formel 1, in welcher
A und B unabhängig voneinander gleich oder verschieden sein können und IIe, Leu, Val, Phe und Tyr bedeuten und C, E, und F unabhängig voneinander gleich oder verschieden sein können und den Rest einer natürlichen Aminosäure außer Cystein und Tryptophan entsprechen und dementsprechend Alanin (Ala), Arginin (Arg), Asparagin (Asn), Asparaginsäure (Asp), Glutamin (Gln), Glutaminsäure (Glu), Glycin (Gly), Histidin (His), Isoleucin (Ile), Leucin (Leu), Lysin (Lys), Methionin (Met), Phenylalanin (Phe), Prolin (Pro), Serin (Ser), Threonin (Thr), Tyrosin (Tyr) oder Valin (Val) sein können, sowie deren physiologisch verträglichen Salze.

Weiterhin bevorzugt sind Verbindungen der Formel 1, die die folgende Peptidsequenz aufweisen: 
cyclo(Val-Val-Xaa-Val-Val-(D)-Ala),
cyclo(Val-Tyr-Xaa-Val-Tyr-(D)-Ala), oder
cyclo(Tyr-Val-Xaa-Tyr-Val-(D)-Ala)
 wobei Xaa den Rest einer natürlichen Aminosäure außer Cys und Trp darstellt.

Unter natürlichen Aminosäuren und deren chemischen Derivate sind alle natürlicherweise frei oder als Bausteine der Proteine auftretenden α-Aminosäuren zu verstehen, wie sie beispielsweise in Practical Handbook of Biochemistry and Molecular Biology, pp. 3-69 (1989) und Gray, Data for Biochemical Res. 2nd ed. pp 1-65 (1969) beschrieben sind.

Im Falle der natürlicherweise in Proteinen vorkommenden Aminosäuren sind unter natürlichen Aminosäuren L-Aminosäuren (mit Ausnahme von Glycin) zu verstehen. (D)-Ala entspricht dem Rest der Aminosäure Alanin in der D-Konfiguration.

Unter Peptiden ist eine Sequenz von Aminosäuren zu verstehen, die durch Peptidbindungen miteinander verknüpft sind, wobei die Peptide einen N-Terminus und einen C-Terminus aufweisen. Die durch Klammern eingerahmten Aminosäuresequenzen beschreiben Cyclopeptide, worin der N-Terminus (die Aminogruppe) der ersten Aminosäure mit dem C-Terminus (der COOH-Gruppe) der letzten Aminosäure verknüpft ist.

Unter physiologisch verträglichen Salzen von Verbindungen der Formel (I) versteht man sowohl anorganische als auch organische Salze, wie sie in Remington's Pharmaceutical Sciences (17. Auflage, Seite 1418 (1985)) beschrieben sind. Als Salze kommen insbesondere Alkali- und Erdalkalisalze, Salze mit physiologisch verträglichen Aminen und Salze mit anorganischen oder organischen Säuren wie z. B. HCI, HBr, H₂SO₄, Maleinsäure, Fumarsäure in Frage.

Bei den oben beschriebenen Verbindungen der Formel (I) handelt es sich um Cyclohexapeptide, die nach allgemein bekannten, in der Peptidchemie üblichen Methoden synthetisiert werden können. Die Synthese der Cyclohexapeptide erfolgt beispielsweise durch
a) Kupplung der geeignet geschützten Aminosäure-Derivate am festen Träger
b) Abspaltung des linearen Peptids unter Erhaltung der Seitenkettenschutzgruppen
c) Cyclisierung des Peptids in Lösung
d) Abspaltung der Seitenkettenschutzgruppen.

Als Aminosäureschutzgruppen sind Schutzgruppen geeignet, wie sie in der Peptidsynthese üblicherweise verwendet werden und beispielsweise in Kontakte Merck 3/79, Seiten 14-22 und 1/80, Seiten 23-35 beschrieben sind. Urethanschutzgruppen der Aminofunktionen sind beispielsweise Pyoc, Fmoc, Fcboc, Z, Boc, Ddz, Bpoc, Z-(NO₂), Dobz, Moc, Mboc, Iboc, Adoc, Adpoc, Msc oder Pioc. Die Entfernung dieser Aminosäureschutzgruppen erfolgt mit Säuren, Basen oder reduktiv.
Schutzgruppen der Guanidinogruppe sind z. B. NO₂, Tosyl, Boc, Z, Mesitylen-2-sulfonyl (Mts) u. ä. Die Abspaltung kann hydrolytisch oder hydrogenolytisch erfolgen.
Die COOH-Seitenfunktionen werden als Alkylester, vorzugsweise Methyl-, Ethyl- oder tert. Butylester oder als Benzylester oder modifizierte Benzylester (p-NO₂, p-Cl, p-Br u.a.) blockiert. Die Deblockierung erfolgt durch alkalische oder saure Verseifung oder Hydrierung.
Hydroxylschutzgruppen sind beispielsweise tert. Butyl oder Benzyl.

Die vorliegende Erfindung betrifft ferner Mischungen der Cyclohexapeptide der allgemeinen Formel (I).

Die Synthese der oben genannten Cyclohexapeptide in Form von Mischungen ist durch Anwendung sogenannter "Kombinatorischer Synthesetechniken" möglich. Kombinatorischer Synthesetechniken, mit deren Hilfe auf einfachen Wege "Peptid Bibliotheken" synthetisiert werden können, sind aus der PCT-Anmeldung WO 92/000091 bekannt.

Die Herstellung von Mischungen der Verbindungen der Formel I kann nach den oben aufgeführten Schritten a-d) erfolgen. Allerdings ist es bei Anwendung kombinatorischer Synthesetechniken nicht nötig, alle möglichen Cyclohexapeptide der Formel I gezielt zu synthetisieren (bei den hier beschriebenen Cyclohexapeptiden sind das immerhin fast 1,9 Millionen (18⁵) verschiedene Verbindungen, bzw. Synthesen). Vielmehr ist es möglich durch gleichzeitige Zugabe aller 18 verschiedener Aminosäuren pro Synthesezyklus eine Mischung von Cyclohexapeptiden der Formel I herzustellen. Der Vorteil dieser Technik gegenüber konventionellen Syntheseverfahren ist evident. So erhält man beispielsweise nach nur 5maligen Durchlaufen eines Synthesezyklus, wobei jeweils alle gewünschten Aminosäuren gleichzeitig eingesetzt werden, und anschließender Cyclisierung mit nur einer Synthese Peptidmischungen, die tatsächlich alle 1, 9 Millionen möglichen Cyclohexapeptide der Formel I enthalten.
Alternativ zum eben beschriebenen Verfahren brauchen aber nicht in allen Synthesezyklen Mischungen der Aminosäuren zugegeben werden. So kann nach einem Synthesezyklus das Trägermaterial, an dem die Petidmischungen synthetisiert wurden, fraktioniert werden und in anschließenden getrennten Synthesereaktionen eine gewünschte Aminosäure an eine bestimmte Position eingefügt werden.

Nach der Herstellung von Peptidmischungen nach den oben beschriebenen Verfahren müssen eventuell biologisch aktive Peptide erkannt und deren Sequenz identifiziert werden. Dies ist durch Anwendung eines iterativen Resyntheseverfahrens möglich, das im folgenden am Beispiel einer Mischung, die Cyclopeptide der Formel A enthält, erläutert wird.

Cyclo(O1-O2-X-X-X-(D)-Ala)          (A)

Diese Mischung enthält Cyclohexapeptide mit drei variablen Positionen (X), die mit allen natürlichen Aminosäuren - außer Cys oder Trp - besetzt sind. Die übrigen Positionen sind mit definierten Aminosäuren (O) (das sind ebenfalls die natürlichen Aminosäuren außer Cys und Trp) besetzt. Eine Position ist immer D-Ala.
Eine Mischung der Cyclopeptide A läßt sich wie oben beschrieben herstellen, indem nach 3 Synthesezyklen unter Verwendung eines Gemisches aller 18 Aminosäuren das Trägermaterial mit den daran gebundenen Peptidmischungen fraktioniert wird und anschließend in 324 (18x18) separaten Synthesen Cyclopeptidmischungen der Formel A hergestellt werden. Jede dieser 324 Mischungen enthält wiederum ca. 5800 (18³) verschiedene Peptide.

Jede der 324 in einem Bioassay (siehe unten) als aktiv erkannte Mischung wird ausgewählt und durch iterative Resynthese unter Neueinfügung einer weiteren definierten Position in Ihrer Komplexizität eingeschränkt. Jede Phase einer iterativen Resynthese erfordert 18 neue Syntheseschritte. Die Wirkung der am Ende erhaltenen Cyclohexapeptide mit definierter Struktur wird durch Testung in einem Bioassay bestätigt. Die eben beschriebene Vorgehensweise wird durch das folgende Schema verdeutlicht.

Peptid mit drei definierten Positionen: Cyclo(O₁-O₂-X-X-X-(D)-Ala)
1. spezifischer Bioassay (324 Tests)
2. Identifikation der aktiven Mischung
3. Synthesen (18) von Peptiden mit 4 definierten Positionen

Peptid mit vier definierten Positionen: Cyclo(O₁-O₂-O₃-X-X-(D)-Ala)
1. spezifischer Bioassay (18 Tests)
2. Identifikation der aktiven Mischung
3. Synthesen (18) von Peptiden mit 5 definierten Positionen

Peptid mit fünf definierten Positionen: Cyclo(O₁-O₂-O₃-O₄-X-(D)-Ala)
1. spezifischer Bioassay (18 Tests)
2. Identifikation der aktiven Mischung
3. Synthesen (18) von Peptiden mit 6 definierten Positionen

Peptid mit sechs definierten Positionen: Cyclo(O₁-O₂-O₃-O₄-O₅-(D)-Ala)
1. spezifischer Bioassay (18 Tests)
2. Identifikation des aktiven Peptids

Prinzipiell lassen sich die Mischungen der Cyclopeptide auch über konventionelle Analysenmethoden wie präparative HPLC oder andere chromatographische Verfahren auftrennen, bzw. aufreinigen.

Es wurde nun gefunden, daß Cyclohexapeptide der allgemeinen Formel I bzw. Mischungen davon spezifisch die Bindung von Interleukin-4 (IL) an den IL-4 Rezeptor inhibieren und die IL-4 Wirkung unterdrücken. Die erfindungsgemäßen Substanzen sind somit als Hemmstoffe der IL-4 Wirkung wirksam.

Die IL-4 inhibierende Wirkung der erfindungsgemäßen Verbindungen bzw. deren Mischungen kann in zellfreien Bindungsassays wie auch in zellulären Bioassays bestimmt werden.

Aus der deutschen Patentanmeldung DE 43 22 330 A1 ist bekannt, daß durch die Unterdrückung der IL-4 Wirkung Krankheiten diagnostiziert, therapiert und/oder behandelt werden können, die durch ein vermehrtes Auftreten von T-Helferzellen vom TH2-Typ gekennzeichnet sind.

Die Therapie und Prophylaxe vieler allergischer, viraler, parasitärer und bakterieller Erkrankungen stellt nach wie vor ein großes Problem dar. Bei einigen parasitären, viralen und bakteriellen Erkrankungen ist bekannt, daß in ihrem Verlauf Veränderungen in Subpopulationen von lymphozytären und monozytären Zellen auftreten. Dies betrifft zum Beispiel das vermehrte Auftreten von sogenannten T-Helfer-Zellen des Typs 2 (im folgenden TH2-Zellen genannt). T-Zellen können generell aufgrund von Oberflächenmarkern und aufgrund ihrer Funktion in Subpopulationen unterteilt werden. So tragen beispielsweise T-Helfer-Lymphozyten CD4-Oberflächenmoleküle und sezernieren nach ihrer Aktivierung Cytokine.

Analysen des Cytokin-Musters von klonierten T-Helferzellen aus gesunden Mäusen oder mit allogenen Zellen stimulierten Mäusen ergaben, daß diese Zellen Interleukin-2, Interleukin-4, Gamma-Interferon, Interleukin-5, Interleukin-6, Interleukin-10 und Lymphotoxin produzieren (T-Helfer Zellen vom sogenannten THO-Typ).

Nach Infektion von Mäusen, zum Beispiel mit dem bakteriellen Antigen Brucella abortus oder mit Mycobacterium tuberkulosis, wurden nach Klonierung von T-Helfer-Zellen vor allem Klone gefunden, die Lymphotoxin, Gamma-Interferon und Interleukin-2, aber wenig oder kein Interleukin-4, Interleukin-5, Interleukin-6 und Interleukin-10 sezernieren (T-Helfer-Zellen vom sogenannten TH1-Typ).

Nach Infektion von beispielsweise suszeptiblen Mäusen mit parasitären Erregern wie Leishmania major traten bei Klonierungen von T-Helferzellen vor allem Klone auf, die vermehrt Interleukin-4, Interleukin-5 und Interleukin-10 produzieren, aber verringerte oder nicht detektierbare Mengen von Interleukin-2 und Gamma-Interferon (T-Helferzellen vom TH2-Typ) (Mosmann et al. Immunological Review 1991, No. 123, 209-229; S. Romagnani, Immunology Today, 256-257; Vol. 12, No. 8 1991).

Dieses vermehrte Auftreten von TH-2-Lymphozyten wurde bereits bei einigen Infektionserkrankungen im Tier und im Menschen nachgewiesen (Else and Grenic, Parasitology Today, Vol. 7, No. 11, 1991, pp. 313-316; Parasitology Today, Vol. 7, No. 10, 1991, p. 261) und spiegelt sich auch in sekundären Parameter wieder. So zeigten mit Leishmania major infizierte Mäuse im allgemeinen eine reduzierte Produktion von Gamma-Interferon, stark erhöhtes Serum IgE und Eosinophilie.

Beim Menschen wurde z.B. bei lepromatöser Lepra, Leishmaniasis und Schistosomiasis und Infektionen mit Mycobacterium tuberculosis im allgemeinen stark erhöhte IgE-Konzentration im Serum dieser Patienten im Vergleich zu Seren von Normalpersonen gefunden. Bei den parasitären Infektionen wird oft eine Eosinophilie im Verlauf der Erkrankung beobachtet.

Auch IgE-vermittelte allergische Reaktionen vom Sofort-Typ wie Atopische Dermatitis und Asthma sind durch eine solche Dysregulation gekennzeichnet. Beispielsweise sind antigen-spezifische T-Zell-Klone aus Hautbiopsien von Patienten mit Atopischer Dermatitis vor allem vom TH2-Typ (Kapsenberg et al. Immunology Today, Vol. 12, No. 11, 1991, 392-395).

Die erfindungsgemäßen Verbindungen und Mischungen eignen sich sowohl zur Therapie und Prophylaxe als auch zur Diagnose von Allergien und Infektionen, insbesondere virale, bakterielle und parasitäre Infektionen sowie Pilzinfektionen; vorzugsweise Infektionen mit dem Humanen Immun-defizienzvirus (HIV), Mycobakterien vor allem Mycobakterium Leprae, mit Listerien, mit Protozoen, vor allem der Gattungen Leishmania und Plasmodium, mit Helminthen, vor allem der Gattung Schistosoma, Nippostronglus und Heligmosomoides mit Trichurida, Trichinella, Taenia (Cysticercus), Candida und Aspergillus. Es können aber auch allergische Reaktionen vom Sofort-Typ, insbesondere IgE-vermittelte Reaktionen diagnostiziert, behandelt oder phrophylaktisch behandelt werden. Vor allem zählen dazu Atopische Dermatitis und Asthma.

Die Applikationsformen sind im allgemeinen bei unterschiedlichen Erkrankungen verschieden. So kann beispielsweise die topische Applikation bei einigen Erkrankungen von Vorteil sein. Zum Beispiel ist bei Asthma eine Inhalationsapplikation, bei Conjuctivitis eine Applikation in Augentropfen, bei Atopischer Dermatitis eine dermale oder intradermale Applikation vorteilhaft, da die pathologischen TH2-Zellen vor allem topisch nachgewiesen werden können.

Die erfindungsgemäßen Peptide bzw. deren Mischungen lassen sich auch allgemein als wissenschaftliche Werkzeuge (Tools) einsetzten, um die Bindung von Interleukin 4 (IL- 4) an IL-4-Rezeptoren zu hemmen.

Die Erfindung betrifft weiterhin pharmazeutische Zubereitungen, die Verbindungen der Formel I oder deren Mischungen enthalten.
Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Die Erfindung betrifft auch ein Verfahren zur Herstellung eines erfindungsgemäßen Arzneimittels, das dadurch gekennzeichnet ist, daß man mindestens eine Verbindung der Formel (I) mit einem pharmazeutisch geeigneten und physiologisch verträglichen Träger und gegebenenfalls weiteren geeigneten Wirk, Zusatz- oder Hilfsstoffen in eine geeignete Darreichungsform bringt.

Bevorzugte Verabreichungsformen sind orale und topische Applikationen, lokale Applikationen wie beispielsweise mit Hilfe eines Katheters oder aber auch Injektionen.

### Experimenteller Teil:

Die folgenden (Herstellungs-) Beispiele sollen der näheren Erläuterung der Erfindung dienen, ohne die Breite der Erfindung in irgendeiner Weise begrenzen zu wollen. Die folgenden Abkürzungen wurden verwendet:

### Abkürzungen:

- AS: Aminosäure
- BSA: Rinderserumalbumin
- DC: Dünnschichtchromatographie
- DCM: Dichlormethan
- DIC: Diisopropylcarbodiimid
- DIPEA: Diisopropylethylamin
- DMF: Dimethylformamid
- DMSO: Dimethylsulfoxid
- ELISA: Enzyme-linked Immuno Sorbent Assay
- Fmoc: 9-Fluorenylmethyloxycarbonyl
- HOBt: 1-Hydroxybenzotriazol
- HPLC: High Pressure (Performance) Liquid Chromatography
- hulL: human Interleukin
- IL: Interleukin
- mulL: murines Interleukin
- PBS: Phosphatpuffersaline
- PBSA: Rinderserumalbumin in PBS
- TBTU: Benzotriazolyl-tetramethyluronium-tetrafluorborat
- TFA: Trifluoressigsäure

Die Abbildungen sind wie folgt beschriftet:
Abbildung 1:
   Kompetitiver hu IL-4 Bindungstest
   ▲ hu IL-4, ● K0021, ■ K0022, ◆K5933
Abbildung 2:
   Kompetitiver mu IL-4 Bindungstest
   ▲ mu IL-4, ● K0021, ■ K0022, ◆K5933
Abbildung 3:
   Kompetitiver hu IL-1ra Bindungstest
   ▲ hu IL-1ra, ● K0021, ■ K0022, ◆K5933
Abbildung 4:
   Austestung der Peptide im hu IL-4 abhängigen Bioassay
   a) Standardkurve hu IL-4 (titriert), ■ hu IL-4
   b) Zusatz von Peptiden (titriert) zu 1 ng/ml hu IL-4 (konstant)
      X K5993, ◆ K0021, ▲K0022
Abbildung 5:
   Austestung der Peptide im mu IL-4 abhängigen Bioassay
   a) Standardkurve mu IL-4 (titriert), ■ mu IL-4
   b) Zusatz von Peptiden (titriert) zu 1 ng/ml mu IL-4 (konstant)
      X K5993, ◆ K0021, ▲K0022
Abbildung 6:
   Austestung der Peptide im hu IL-1 beta abhängigen Bioassay
   a) Standardkurve hu IL-1 beta (titriert), ■ hu IL-1 beta
   b) Zusatz von Peptiden (titriert) zu 0,5 U/ml hu IL-1 beta (konstant)
      X K5993, ◆ K0021, ▲K0022

### Synthese der Cyclohexapeptide

Die beschriebenen Cyclopeptide werden als Einzelpeptide oder in Mischung hergestellt. Die Synthese erfolgt durch
a) Kupplung der geeignet geschützten Aminosäure-Derivate am festen Träger
b) Abspaltung des linearen Peptids unter Erhaltung der Seitenkettenschutzgruppen
c) Cyclisierung des Peptids in Lösung
d) Abspaltung der Seitenkettenschutzgruppen

### a) Synthese der linearen Peptide oder Peptidmischungen

13.1 g Fmoc-D-Ala-Tritylharz werden in 131 ml Dichlormethan (DCM/Dimethylformamid) (DMF) (2:1) suspendiert und jeweils 0.4 ml (40 mg, 0.032 mmol) der Harz-Suspension werden in die vorbereiteten Reaktionsgefäße pipettiert. Als Reaktionsgefäße werden Eppendorfspitzen mit Glaswolle gestopft und in die Syntheseblöcke eines multiplen Peptidsyntheseautomaten gesteckt. Es werden 0.7 M Lösungen der benötigten Fmoc-Aminosäuren angesetzt, die Kupplung erfolgt mit 5-fachem Überschuß an Fmoc-Aminosäure nach in situ Aktivierung mit Diisopropylcarbodiimid (DIC), welches als 1.5 M Lösung in DMF/DCM (1:2) in 5-fachem Überschuß zugegeben wird. Die Kupplungszeit beträgt 50 min.

Definierte Peptidmischungen werden durch Mischungen der Trägerstoffe (polymeren beads) erhalten, die mit Einzelpeptiden belegt sind.

Protokoll des Synthesezyklus am SYRO-Syntheseautomaten:

| Operation | Reagenz/Solvens | Vol. [µl] | Zeit |
|---|---|---|---|
| 1. Quellen | DMF | 400 | 2 x 1 min |
| 2. Deblockierung | 40 % Piperidin/DMF | 250 | 1 x 15 min |
| 3. Waschen | DMF | 400 | 7 x 0.8 min |
| 4. Kupplung | Fmoc-AS/HOBT (0.7 M) | 220 | 50 min |
| | DIC (1.7 M) | 100 | |
| 5. Waschen | DMF | 230/250/300 | 3 x 0.8 min |

### b) Abspaltung der linearen Peptide oder Peptidmischungen von Harz

Die Abspaltung der Peptide bzw. Peptidmischungen vom Harz (je 1g) erfolgt in sog. Falcons® mit 30 ml Essigsäure/Methanol/DCM (2:2:6), 3 h unter Schütteln bei Raumtemperatur. Die Harze werden von der Abspaltlösung abfiltriert, und die Lösungsmittel werden im Vakuumskonzentrator bei 300 mbar/40°C (DCM/Methanol) bzw. 1 mbar/40°C (Essigsäure) entfernt. Die Rückstände werden in tert. Butylalkohol/Wasser (4:1) gelöst, mit 2 eq 0.2 N HCI (bezogen auf die Aminogruppen) versetzt und im Vakuumskonzentrator abermals eingeengt.

### c) Cyclisierung der linearen Peptide oder Peptidmischungen

Es werden 0.4 mmol eines Peptids oder einer Peptidmischung in 250 ml DMF (0.0016 M) in Polypropylenflaschen gelöst und mit 4 eq Diisopropylethylamin (DIPEA) versetzt. Unter Schütteln werden je 3 ml einer 0.4 M HOBt/TBTU Lösung in DMF (3 eq, 1.2 mmol) langsam zugetropft. Mit DC wird der Reaktionsverlauf kontrolliert und die Reaktion nach 5 h beendet. Die Lösungsmittel werden über Nacht (15 h) bei 1 mbar/40°C im Vakuumskonzentrator entfernt, die trockenen Rückstände werden in DCM aufgenommen und mit 5 % KHSO₄, 5% NaHCO₃-Lösungen und Wasser ausgeschüttelt (je 3x). Die organischen Phasen werden über Na₂SO₄ getrocknet, filtriert und im Vakuum eingeengt. Die Rückstände wurden in tert.-Butylalkohol/Wasser (4:1) gelöst und lyophilisiert.

### d) Abspaltung der Seitenkettenschutzgruppen

Je 100-200 mg des Cyclopeptids oder der Cvclopeptidmischung werden mit 5 bzw. 10 ml Abspaltlösung (TFA/Thioanisol/Thiocresol, 0.95:0.25:0.25) 4 h bei Raumtemperatur behandelt, um die Seitenkettenschutzgruppen abzuspalten. Anschließend werden die Cyclopeptide in Diethylether/n-Heptan (1:1) ausgefällt, zentrifugiert, noch zweimal mit Diethylether/n-Heptan gewaschen, in tert.-Butylalkohol/Wasser (4:1) gelöst und gefriergetrocknet.

### Beispiel 1

### Synthese von cyclo(Val-Tyr-Xaa-Val-Tyr-(D)-Ala) (K0021)

### a) Synthese der linearen Peptidmischung cyclo(Val-Tyr-Xaa-Val-Tyr-(D)-Ala)

Zur Synthese der linearen Peptidmischung (Val-Tyr-Xaa-Val-Tyr-(D)-Ala) bestehend aus 18 Komponenten (Val-Tyr-Ala-Val-Tyr-(D)-Ala; Val-Tyr-Arg-Val-Tyr-(D)-Ala; Val-Tyr-Asn-Val-Tyr-(D)-Ala; Val-Tyr-Asp-Val-Tyr-(D)-Ala; Val-Tyr-Gln-Val-Tyr-(D)-Ala; Val-Tyr-Glu-Val-Tyr-(D)-Ala; Val-Tyr-Gly-Val-Tyr-(D)-Ala; Val-Tyr-His-Val-Tyr-(D)-Ala; Val-Tyr-lle-Val-Tyr-(D)-Ala; Val-Tyr-Leu-Val-Tyr-(D)-Ala; Val-Tyr-Lys-Val-Tyr-(D)-Ala; Val-Tyr-Met-Val-Tyr-(D)-Ala; Val-Tyr-Phe-Val-Tyr-(D)-Ala; Val-Tyr-Pro-Val-Tyr-(D)-Ala; Val-Tyr-Ser-Val-Tyr-(D)-Ala; Val-Tyr-Thr-Val-Tyr-(D)-Ala; Val-Tyr-Tyr-Val-Tyr-(D)-Ala; Val-Tyr-Val-Val-Tyr-(D)-Ala) werden diese als Einzelpeptide an polymeren Trägern mit Hilfe eines Roboters zur multiplen Peptidsynthese hergestellt. Als Reaktionsgefäße werden Filtersäulen aus Polypropylen mit je 40 mg (= 0.032 mmol) Fmoc-D-Ala-2-Chlortrityl-Harz gefüllt. Das Verteilen der Harzmengen erfolgt entweder durch Einwaage des trockenen Harzes oder durch Pipettieren einer Harzsuspension in Dichlormethan/Dimethylformamid (2:1). Es werden 0.7 M Lösungen der benötigten Fmoc-Aminosäuren mit äquimolaren Mengen N-Hydroxybenzotriazol (HOBt) in Dimethylformamid (DMF) angesetzt, die Kupplung erfolgt mit 5-fachem Überschuß an Fmoc-Aminosäuren durch in situ Aktivierung mit DIC, welches als 1.5 M Lösung in Dichlormethan/Dimethylformamid (2:1) in 5-fachem Überschuß zugegeben wird. Die Kupplungszeit beträgt 50 min.

### b) Abspaltung der linearen Peptidmischung vom Harz

18 einzelne Hexapeptid-Harze werden vor der Abspaltung der Peptide vom Harz zu der Peptidmischung (Val-Tyr-Xaa-Val-Tyr-(D)-Ala) vereinigt (18 x 40 mg = 0.72 g Peptidharz). Die Abspaltung der Peptidmischung von insgesamt 0.72 g Peptidharz erfolgt mit 20 ml Essigsäure/Methanol/Dichlormethan (2:2:6), 3 h unter Schütteln bei Raumtemperatur. Das Harz wird von der Abspaltlösung abfiltriert und das Lösungsmittel in einem Rotationsvakuumkonzentrator (Beta-RVC, Christ, Osterode) bei 200 mbar/40°C (DCM/Methanol) bzw. 1 mbar/40°C (Essigsäure) entfernt. Der Rückstand wird in tert. Butylalkohol/Wasser (4:1) gelöst, mit 2 eq 0.2 N HCl (bezogen auf die Aminogruppe) versetzt und im Rotationsvakuumkonzentrator bis zur Trockene eingeengt.

### c) Cyclisierung

Peptidmischung (Val-Tyr-Xaa-Val-Tyr-(D)-Ala) (0.4 mmol) wird in 250 ml DMF (0.0016 M) in einer Polypropylenflasche gelöst, mit 4 eq Diisopropylethylamin (DIPEA) versetzt und 1 h im Gefrierschrank gekühlt. Unter Schütteln werden 3 ml einer 0.4 M HOBt/TBTU/DMF-Lösung (= 3 eq, 1.2 mmol) langsam zugetropft. Der Reaktionsverlauf wird durch Dünnschichtchromatographie kontrolliert (Fließmittel: Chloroform/Methanol/Eisessig 85:15:2, R_{F Produkt} = 0.59). Die Reaktion ist nach 3 h beendet. Das Lösungsmittel wird bei 1 mbar/40°C im Rotationsvakuumkonzentrator entfernt, der trockene Rückstand wird in Dichlormethan gelöst und mit 5 % KHSO₄-Lösung und Wasser ausgeschüttelt. Die organische Phase wird über Na₂SO₄ getrocknet. Das Lösungsmittel wird im Vakuum abgedampft und der Rückstand wird in tert. Butylalkohol/Wasser (4:1) gelöst und lyophilisiert.

### d) Abspaltung der Seitenkettenschutzgruppen

Cyclopeptidmischung (Val-Tyr-Xaa-Val-Tyr-(D)-Ala)(100-200 mg) wird mit 10 ml Abspaltlösung (TFA/Thioanisol/Thiocresol, 0.95:0.025:0.025) 4 h bei Raumtemperatur behandelt, um die Seitenkettenschutzgruppen abzuspalten. Die Abspaltungslösung wird unter Rühren langsam zu Diethylether/n-Heptan (1:1) zugegeben, das Präzipitat wird abzentrifugiert und das Sediment wird zweimal mit Diethylether/n-Heptan durch Behandlung mit Ultraschall gewaschen, in tert. Butylalkohol/Wasser (4:1) gelöst und gefriergetrocknet.

### e) Analytik der Cyclopeptidmischung

Die Analytik der Cyclopeptidmischung erfolgt durch HPLC und Ionenspray-Massenspektroskopie. MS (FAB): cyclo(Val-Tyr-Xaa-Val-Tyr-(D)-Ala); Xaa = Gly: 653,8 (M+H); Xaa = Ala: 667,8 (M+H); Xaa = Ser: 683,8 (M+H); Xaa = Pro: 693,9 (M+H); Xaa = Val: 695,9 (M+H); Xaa = Thr: 697,9 (M+H); Xaa = Leu: 709,9 (M+H); Xaa = Ile: 709,9 (M+H); Xaa = Asn: 710,9 (M+H); Xaa = Asp: 711,8 (M+H); Xaa = Lys: 724,9 (M+H); Xaa = Gln: 724,9 (M+H); Xaa = Glu: 725,9 (M+H); Xaa = Met: 728 (M+H); Xaa = His: 733,9 (M+H); Xaa = Phe: 743,9 (M+H); Xaa = Arg: 752,9 (M+H); Xaa = Tyr: 759,9 (M+H).

### Beispiel 2

### Synthese von cyclo(Val-Val-Xaa-Val-Val-(D)-Ala) (K 5993)

Die Synthese der Cyclopeptidmischung cyclo(Val-Val-Xaa-Val-Val-(D)-Ala) erfolgt analog dem Beispiel 1 unter Verwendung der entsprechenden Aminosäuren. MS(FAB): cyclo(Val-Val-Xaa-Val-Val-(D)-Ala); Xaa = Gly: 525,7 (M+H); Xaa = Ala: 539,7 (M+H); Xaa = Ser: 555,7 (M+H); Xaa = Pro: 565,8 (M+H); Xaa = Val: 567,8 (M+H); Xaa = Thr: 569,8 (M+H); Xaa = Leu: 581,8 (M+H); Xaa = Ile: 581,8 (M+H); Xaa = Asn: 582,8 (M+H); Xaa = Asp: 583,7 (M+H); Xaa = Lys: 596,8 (M+H); Xaa = Gln: 596,8 (M+H); Xaa = Glu: 597,8 (M+H); Xaa = Met: 599,9 (M+H); Xaa = His: 605,8 (M+H); Xaa = Phe: 615,8 (M+H); Xaa = Arg: 624,8 (M+H); Xaa = Tyr: 631,8 (M+H).

### Beispiel 3

### Synthese von cyclo(Tyr-Val-Xaa-Tyr-Val-(D)-Ala) (K 0022)

Die Synthese von Cyclopeptidmischung cyclo(Tyr-Val-Xaa-Tyr-Val-(D)-Ala) erfolgt analog dem Beispiel 1 unter Verwendung der entsprechenden Aminosäuren. MS (FAB): cyclo(Tyr-Val-Xaa-Tyr-Val-(D)-Ala); Xaa = Gly: 653,8 (M+H); Xaa = Ala: 667,8 (M+H); Xaa = Ser: 683,8 (M+H); Xaa = Pro: 693,9 (M + H); Xaa = Val: 695,9 (M + H); Xaa = Thr: 697,9 (M+H); Xaa = Leu: 709,9 (M+H); Xaa = Ile: 709,9 (M+H); Xaa = Asn: 710,9 (M+H); Xaa = Asp: 711,8 (M + H); Xaa = Lys: 724,9 (M+H); Xaa = Gln: 724,9 (M+H); Xaa = Glu: 725,9 (M+H); Xaa = Met: 728 (M+H); Xaa = His: 733,9 (M+H); Xaa = Phe: 743,9 (M+H); Xaa = Arg: 752,9 (M+H); Xaa = Tyr: 759,9 (M+H).

### Beispiel 4

Die folgenden Cyclopeptide bzw. Cyclopeptidgemische wurden analog hergestellt.

cyclo(Val-Tyr-Ala-Val-Tyr-(D)-Ala)
cyclo(Tyr-Val-Ala-Tyr-Val-(D)-Ala)
cyclo(Tyr-Val-Hyp-Tyr-Val-(D)-Ala)
cyclo(Tyr-Val-Ala-Gly-Xaa-(D)-Ala)
cyclo(Tyr-Val-Ala-Gln-Xaa-(D)-Ala)
cyclo(Tyr-Val-Ala-Lys-Xaa-(D)-Ala)
cyclo(Tyr-Val-Ala-Asn-Xaa-(D)-Ala)
cyclo(Tyr-Val-Ala-Ile-Xaa-(D)-Ala)
cyclo(Tyr-Val-Ala-Leu-Xaa-(D)-Ala)
cyclo(Tyr-Val-Ala-Pro-Xaa-(D)-Ala)
cyclo(Tyr-Val-Ala-Phe-Xaa-(D)-Ala)
cyclo(Tyr-Val-Ala-His-Xaa-(D)-Ala)
cyclo(Tyr-Val-Ala-Ala-Xaa-(D)-Ala)
cyclo(Tyr-Val-Ala-Tyr-Xaa-(D)-Ala)
cyclo(Tyr-Val-Gln-Tyr-Val-(D)-Ala)
cyclo(Tyr-Val-Lys-Tyr-Val-(D)-Ala)
cyclo(Tyr-Val-Glu-Tyr-Val-(D)-Ala)
cyclo(Tyr-Val-Asp-Tyr-Val-(D)-Ala)
cyclo(Tyr-Val-Phe-Tyr-Val-(D)-Ala)
cyclo(Tyr-Val-Gly-Tyr-Val-(D)-Ala)

cyclo(Tyr-Val-His-Tyr-Val-(D)-Ala)
cyclo(Tyr-Val-Ile-Tyr-Val-(D)-Ala)
cyclo(Tyr-Val-Leu-Tyr-Val-(D)-Ala)
cyclo(Tyr-Val-Asn-Tyr-Val-(D)-Ala)
cyclo(Tyr-Val-Pro-Tyr-Val-(D)-Ala)
cyclo(Tyr-Val-Arg-Tyr-Val-(D)-Ala)
cyclo(Tyr-Val-Ser-Tyr-Val-(D)-Ala)
cyclo(Tyr-Val-Thr-Tyr-Val-(D)-Ala)
cyclo(Tyr-Val-Val-Tyr-Val-(D)-Ala)
cyclo(Tyr-Val-Tyr-Tyr-Val-(D)-Ala)
cyclo(Tyr-Val-Met-Tyr-Val-(D)-Ala)

Hyp steht für Hydroxyprolin

### Messung der biologischen Aktivität in Bioassays

### Beispiel 5:

### Spezifische Inhibition der IL-4 Bindung in zellfreien Bindungsassays

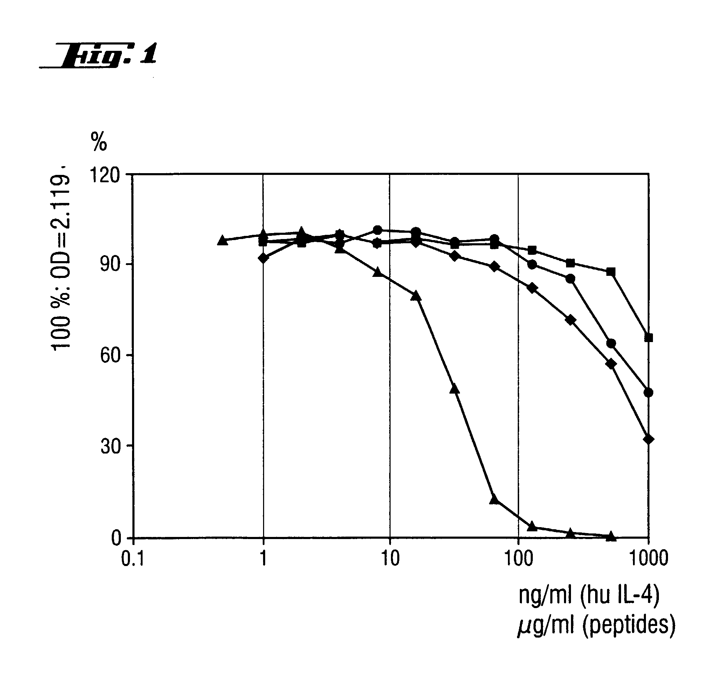

Aus der EP 488 170 A1 sind zellfreie Bindungsteste bekannt. Für ihre Durchführung werden rekombinante chimäre Proteine verwendet, die aus der extrazellulären Region von normalerweise membranständigen Rezeptoren bestehen, an deren Carboxyterminus die Fc-Region einer schweren Immunglobulinkette bestehend aus Hinge-, CH2- und CH3-Domäne fusioniert wurde. Diese sogenannten Rezeptor/Fc-Fusionsproteine können unter Erhalt der spezifischen Rezeptorbindungsaktivität an Festphasen gebunden werden, die beispielsweise mit monoklonalen Antikörpern gerichtet gegen den Fc-Teil vorbeschichtet wurden. Als Festphase wurden für dieses Beispiel ®Nunc Typ B ELISA-Platten verwendet. Rezeptor/Fc-Fusionsproteine gelöst in PBS enthaltend 10 mg/ml BSA (PBSA) wurden an diese vorbehandelten Platten gebunden (HulL-4R/Fc: 500 ng/ml; mulL-4R/Fc: 250 ng/ml; huIL-1R/Fc: 125 ng/ml; 1 h bei Raumtemperatur). Nach Waschen wurden die Peptide beziehungsweise die jeweiligen spezifischen unmarkierten Liganden (huIL-4R/Fc: huIL-4; muIL-4R/Fc: muIL-4; huIL-1R/Fc:huIL-1ra) in variierenden Konzentrationen und danach die spezifischen Liganden in biotinylierender Form in einer festen Konzentration zugegeben (100 ng/ml). Die Inkubation erfolgte 1 h bei Raumtemperatur in PBSA enthaltend 5 % DMSO. Nach Waschen erfolgte eine Inkubation mit Streptavidin/Peroxidase (Amersham, 1:2000 in PBSA) für 20 min bei Raumtemperatur. Der Nachweis der gebundenen Peroxidase geschah nach abermaligen Waschen in einer Tetramethylbenzidin-Substratlösung (Behringwerke). Nach 30 min Inkubation wurde die Extinktion bei 450 nm gemessen, die direkt die Menge des an den Rezeptor gebundenen biotinylierter Liganden reflektiert. Für die Abbildung 1-3 wurde das gemessene Extinktionssignal in Abhängigkeit von der Konzentration des kompetierenden Agens aufgetragen, wobei als 100 %-Wert die gemessene Extinktion in Abwesenheit eines kompetierenden Agens herangezogen wurde.

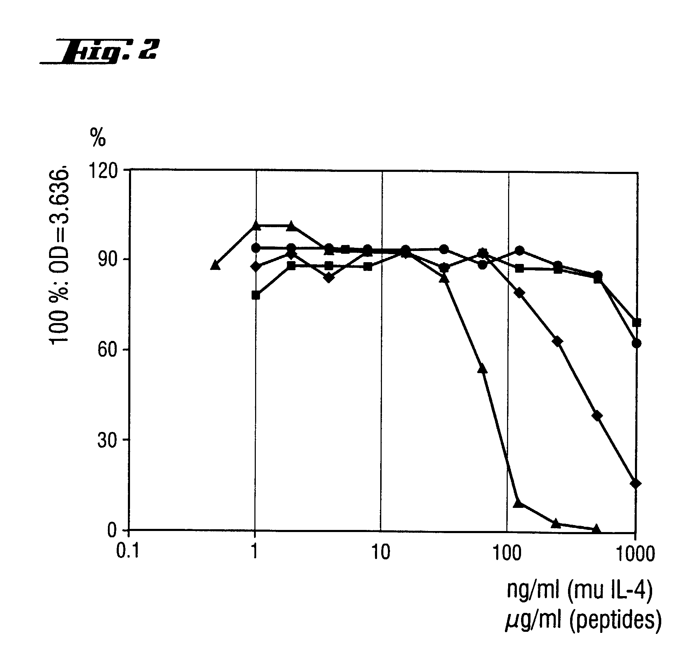

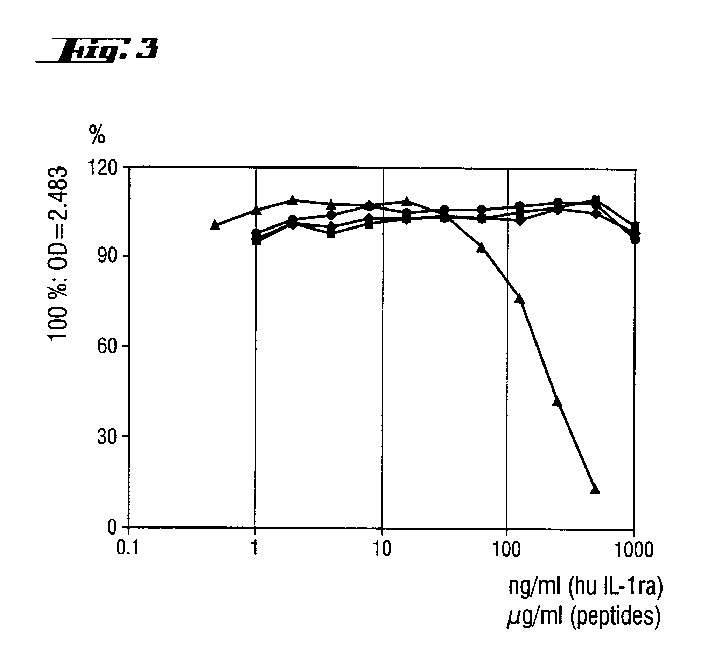

Abb. 1 zeigt, daß im huIL-4 Bindungstest unmarkiertes huIL-4 mit biotinyliertem huIL-4 um die Bindung an den humanen IL-4 Rezeptor kompetiert. Entsprechendes gilt für muIL-4 und murinen IL-4 Rezeptor (Abb. 2) sowie für huIL-1ra und humanen IL-1-Rezeptor (Abb. 3). Die Peptidmischungen K 5993, K 0021 und K 0022 hingegen inhibieren die Bindung von biotinylierten huIL-4 and muIL-4 an ihre jeweiligen Rezeptoren (Abb. 1 und 2), während sie im IL-1ra-Bindungsassay keinen signifikanten Effekt bewirken (Abb.3).
Folgende IC₅₀-Werte wurden ermittelt:

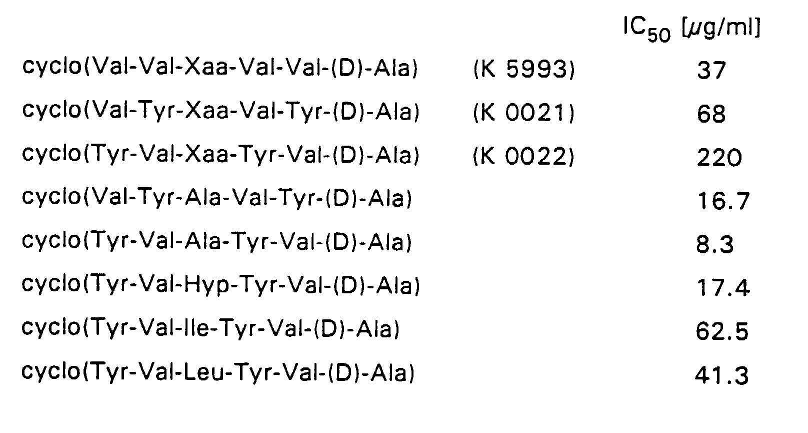

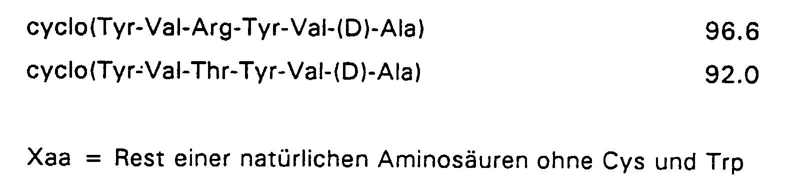

### Beispiel 6:

### Einfluß auf die IL-4 induzierte Proliferation

Die biologische Aktivität der Cyclohexapeptide der Formel I bzw. deren Mischungen wurde in einem Bioassay gemessen. IL-4 bindet spezies-spezifisch an den IL-4 Rezeptor. Aus diesem Grund wurde eine Zellinie verwendet, die murinen Ursprungs ist und den murinen IL-4 Rezeptor membranständig trägt. Diese Zellinie wurde mit dem vollständigen Gen für humanen Interleukin-4 Rezeptor transfiziert. Murine und humane membranständige Rezeptoren werden gleichzeitig funktionell von dieser Zellinie exprimiert und die Zellinie proliferiert sowohl in Abhängigkeit von murinen, als auch humanen IL-4 (Mosley et al.; Cell, Vol. 59, 335-348, October 20, 1989). Zur besseren Löslichkeit der Peptide wurde allen Kulturmedien 1 % DMSO zugesetzt.

### a) Einfluß auf die durch humanes IL-4 induzierte Proliferation

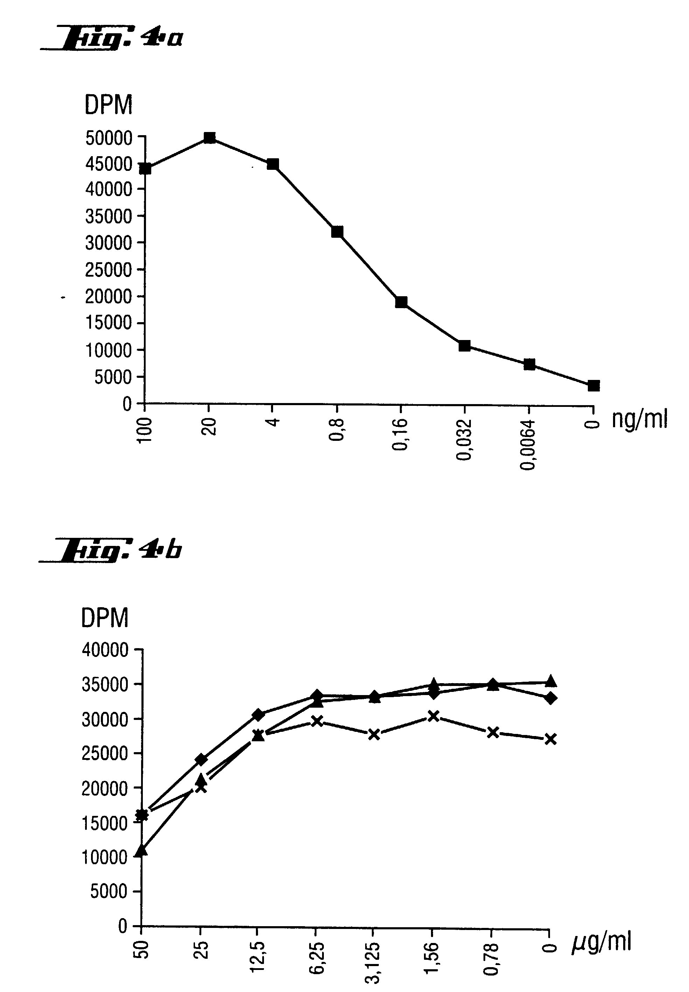

Die Zellinie proliferiert in Abhängigkeit von humanen IL-4 (hu IL-4), dargestellt in Abb. 4 (Standardkurve). Bei Verwendung einer konstanten Konzentration von huIL-4 kommt es zur konzentrationsabhängigen Hemmung der Proliferation durch die jeweils angegebenen, einzeln zutitrierten Peptidmischungen (Abb. 4).

### b) Einfluß auf die durch murines IL-4 induzierte Proliferation

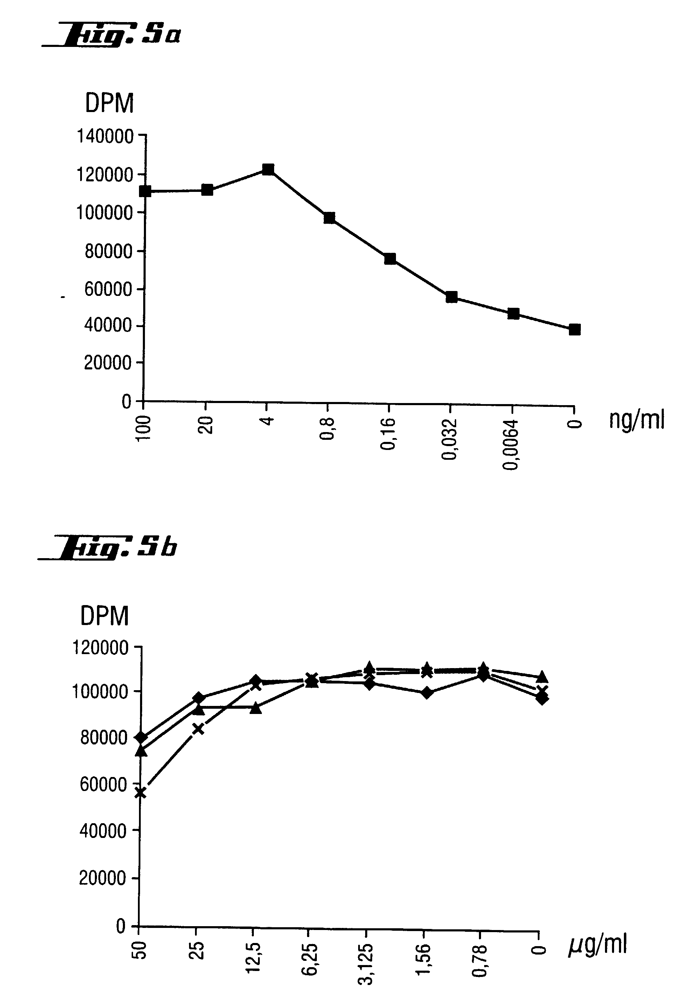

Die Zellinie proliferiert in Abhängigkeit von murinem IL-4 (mu IL-4), dargestellt in Abb. 5 (Standardkurve). Bei Verwendung einer konstanten Konzentration von muIL-4 kommt es zu einer konzentrationsabhängigen Hemmung der Proliferation durch die jeweils angegebenen, einzeln zutitrierten Peptidmischungen (Abb. 5).

### Beispiel 7:

### Einfluß auf die IL-1 induzierte Proliferation

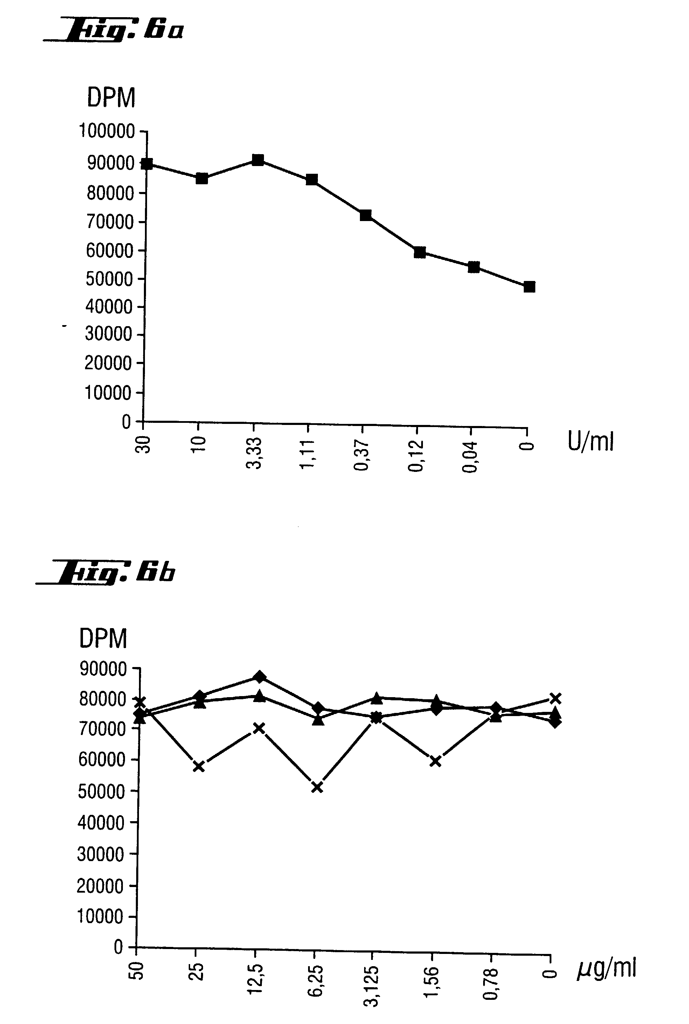

Verwendet wurde ein IL-1 abhängiger T-Zell Klon (D10(N4)M, Hopkins et al., J. of Imm. Methods, 120, 271-276, 1989). Dem Kulturmedium war zur besseren Löslichkeit der Peptide 1 % DMSO zugesetzt. Die Zellen proliferieren in Abhängigkeit von humanen IL-1 beta (hu IL-1 beta) (Abb. 6, Standardkurve). Bei Verwendung einer konstanten Konzentration von hu IL-1 beta kommt es zu keiner Hemmung der Proliferation der Zellinie durch die jeweils angegebenen, einzeln zutitrierten Peptidmischungen im angegebenen Konzentrationsbereich (Abb. 6).

## Patentansprüche

1. Verbindung der Formel I cyclo(A-B-C-E-F-(D)-Ala)          (I) in welcher A und B gleich oder verschieden sein können und Ile, Leu, Val, Phe oder Tyr bedeuten und C, E, und F gleich oder verschieden sein können und den Rest einer natürlichen Aminosäure außer Cystein und Tryptophan bedeuten,
sowie deren physiologisch verträglichen Salze.

2. Verbindung der Formel I gemäß Anspruch 1, in welcher A und B gleich oder verschieden sein können und Ile, Leu, Val, Phe oder Tyr bedeuten und C, E, und F gleich oder verschieden sein können und Ala, Arg, Asp, Asn, Gln, Glu, Gly, His, Ile, Leu, Lys, Met, Phe, Pro, Ser, Thr, Tyr oder Val bedeuten,
sowie deren physiologisch verträglichen Salze.

3. Verbindung der Formel 
cyclo(Val-Val-Xaa-Val-Val-(D)-Ala);
cyclo(Val-Tyr-Xaa-Val-Tyr-(D)-Ala) oder
cyclo(Tyr-Val-Xaa-Tyr-Val-(D)-Ala);
 wobei Xaa den Rest einer natürlichen Aminosäure außer Cystein und Tryptophan bedeutet,
sowie deren physologisch verträglichen Salze.

4. Verbindung der Formel 
cyclo(Tyr-Val-Ala-Gly-Xaa-(D)-Ala);
cyclo(Tyr-Val-Ala-Gln-Xaa-(D)-Ala);
cyclo(Tyr-Val-Ala-Lys-Xaa-(D)-Ala);
 
cyclo(Tyr-Val-Ala-Asn-Xaa-(D)-Ala);
cyclo(Tyr-Val-Ala-Ile-Xaa-(D)-Ala);
cyclo(Tyr-Val-Ala-Leu-Xaa-(D)-Ala);
cyclo(Tyr-Val-Ala-Pro-Xaa-(D)-Ala);
cyclo(Tyr-Val-Ala-Phe-Xaa-(D)-Ala);
cyclo(Tyr-Val-Ala-His-Xaa-(D)-Ala);
cyclo(Tyr-Val-Ala-Ala-Xaa-(D)-Ala) oder
cyclo(Tyr-Val-Ala-Tyr-Xaa-(D)-Ala);
 wobei Xaa den Rest einer natürlichen Aminosäure außer Cystein und Tryptophan bedeutet,
sowie deren physiologisch verträglichen Salze.

5. Verbindung der Formel 
cyclo(Val-Tyr-Ala-Val-Tyr-(D)-Ala);
cyclo(Val-Tyr-Arg-Val-Tyr-(D)-Ala);
cyclo(Val-Tyr-Asn-Val-Tyr-(D)-Ala);
cyclo(Val-Tyr-Asp-Val-Tyr-(D)-Ala);
cyclo(Val-Tyr-Gln-Val-Tyr-(D)-Ala);
cyclo(Val-Tyr-Glu-Val-Tyr-(D)-Ala);
cyclo(Val-Tyr-Gly-Val-Tyr-(D)-Ala);
cyclo(Val-Tyr-His-Val-Tyr-(D)-Ala);
cyclo(Val-Tyr-Ile-Val-Tyr-(D)-Ala);
cyclo(Val-Tyr-Leu-Val-Tyr-(D)-Ala);
cyclo(Val-Tyr-Lys-Val-Tyr-(D)-Ala);
cyclo(Val-Tyr-Met-Val-Tyr-(D)-Ala);
cyclo(Val-Tyr-Phe-Val-Tyr-(D)-Ala);
cyclo(Val-Tyr-Pro-Val-Tyr-(D)-Ala);
cyclo(Val-Tyr-Ser-Val-Tyr-(D)-Ala);
cyclo(Val-Tyr-Thr-Val-Tyr-(D)-Ala);
cyclo(Val-Tyr-Tyr-Val-Tyr-(D)-Ala) oder
cyclo(Val-Tyr-Val-Val-Tyr-(D)-Ala);
 sowie deren physiologisch verträglichen Salze.

6. Verbindung der Formel 
cyclo(Val-Tyr-Ala-Val-Tyr-(D)-Ala);
cyclo(Tyr-Val-Ala-Tyr-Val-(D)-Ala);
cyclo(Tyr-Val-Hyp-Tyr-Val-(D)-Ala);
cyclo(Tyr-Val-Gln-Tyr-Val-(D)-Ala);
cyclo(Tyr-Val-Lys-Tyr-Val-(D)-Ala);
cyclo(Tyr-Val-Glu-Tyr-Val-(D)-Ala);
cyclo(Tyr-Val-Asp-Tyr-Val-(D)-Ala);
cyclo(Tyr-Val-Phe-Tyr-Val-(D)-Ala);
cyclo(Tyr-Val-Gly-Tyr-Val-(D)-Ala);
cyclo(Tyr-Val-His-Tyr-Val-(D)-Ala);
cyclo(Tyr-Val-Ile-Tyr-Val-(D)-Ala);
cyclo(Tyr-Val-Leu-Tyr-Val-(D)-Ala);
cyclo(Tyr-Val-Asn-Tyr-Val-(D)-Ala);
cyclo(Tyr-Val-Pro-Tyr-Val-(D)-Ala);
cyclo(Tyr-Val-Arg-Tyr-Val-(D)-Ala);
cyclo(Tyr-Val-Ser-Tyr-Val-(D)-Ala);
cyclo(Tyr-Val-Thr-Tyr-Val-(D)-Ala);
cyclo(Tyr-Val-Val-Tyr-Val-(D)-Ala);
cyclo(Tyr-Val-Tyr-Tyr-Val-(D)-Ala) oder
cyclo(Tyr-Val-Met-Tyr-Val-(D)-Ala);
 sowie deren physiologisch verträglichen Salze.

7. Verfahren zur Herstellung einer Verbindung der Formel I gemäß einem oder mehreren der Ansprüche 1-6, dadurch gekennzeichnet, daß man
a) geeignet geschützte Aminosäurederivate an einen festen Träger kuppelt,
b) das gebildete lineare Peptid unter Erhaltung der Seitenkettenschutzgruppen vom Träger abspaltet,
c) das lineare Peptid in Lösung cyclisiert,
d) die Seitenkettenschutzgruppen abspaltet, und gegebenenfalls
e) in ein physiologisch verträgliches Salz überführt.

8. Mischung, enthaltend Verbindungen gemäß einem oder mehreren der Ansprüche 1-6.

9. Verfahren zur Herstellung einer Mischung gemäß Anspruch 8, dadurch gekennzeichnet, daß man
a) geeignet geschützte Aminosäurederivate an einen festen Träger kuppelt, wobei zur Einführung variabler Positionen entweder eine Mischung aller gewünschten Aminosäuren hinzugegeben wird, oder der Träger mit den daran gekuppelten Oligopeptiden in Fraktionen aufteilt wird und jede Fraktion einzeln mit einer spezifischen Aminosäure gekuppelt wird und anschließend die Fraktionen wieder vereinigt werden,
b) die gebildete Mischung linearer Peptide unter Erhaltung der Seitenkettenschutzgruppen vom Träger abspaltet,
c) die Mischung linearer Peptide in Lösung cyclisiert, und
d) die Seitenschutzgruppen abspaltet, und gegebenenfalls
e) in ein physiologisch verträgliches Salz überführt.

10. Verwendung einer Verbindung der Formel I gemäß einem oder mehreren der Ansprüche 1-6 zur Herstellung eines Medikaments.

11. Verwendung einer Verbindung der Formel I gemäß einem oder mehreren der Ansprüche 1-6 zur Herstellung eines Medikaments zur Behandlung oder Prophylaxe von Allergien und Infektionen.

12. Verwendung einer Verbindung der Formel I gemäß einem oder mehreren der Ansprüche 1-6 zur Herstellung eines Diagnostikums.

13. Verwendung einer Verbindung der Formel I gemäß einem oder mehreren der Ansprüche 1-6 zur Herstellung eines wissenschaftlichen Tools zur Hemmung der Bindung von Interleukin-4 (IL-4) an den IL-4-Rezeptor.

14. Arzneimittel oder Diagnostikum, enthaltend eine wirksame Menge einer Verbindung der Formel I gemäß einem oder mehreren der Ansprüche 1-6.

15. Verfahren zur Herstellung eines Arzneimittels oder Diagnostikums gemäß Anspruch 14, dadurch gekennzeichnet, daß mindestens eine Verbindung nach einem oder mehreren der Ansprüche 1-6 mit einem physiologisch annehmbaren Träger sowie gegebenenfalls geeigneten Zusatzstoffen und/oder Hilfsstoffen vermischt wird.

16. Verwendung einer Mischung gemäß Anspruch 8 zur Herstellung eines Medikaments zur Behandlung oder Prophylaxe von Allergien und Infektionen.

17. Verwendung einer Mischung gemäß Anspruch 8 zur Herstellung eines Diagnostikums.

18. Verwendung einer Mischung gemäß Anspruch 8 zur Herstellung eines wissenschaftlichen Tools zur Hemmung der Bindung von Interleukin-4 (IL-4) an den IL-4-Rezeptor.

19. Arzneimittel oder Diagnostikum, enthaltend eine wirksame Menge einer Mischung gemäß Anspruch 8.

20. Verfahren zur Herstellung eines Arzneimittels oder Diagnostikums gemäß Anspruch 19, dadurch gekennzeichnet, daß eine oder mehrere Mischungen gemäß Anspruch 8 mit einem physiologisch annehmbaren Träger sowie gegebenenfalls geeigneten Zusatzstoffen und/oder Hilfsstoffen vermischt wird.

## Claims

1. A compound of the formula I cyclo(A-B-C-E-F-(D)-Ala)          (I) in which A and B can be identical or different and are Ile, Leu, Val, Phe or Tyr and C, E and F can be identical or different and are the residue of a natural amino acid other than cysteine and tryptophan,
and the physiologically tolerated salts thereof.

2. A compound of the formula I as claimed in claim 1, in which A and B can be identical or different and are Ile, Leu, Val, Phe or Tyr and C, E and F can be identical or different and are Ala, Arg, Asp, Asn, Gln, Glu, Gly, His, Ile, Leu, Lys, Met, Phe, Pro, Ser, Thr, Tyr or Val,
and the physiologically tolerated salts thereof.

3. A compound of the formula 
cyclo(Val-Val-Xaa-Val-Val-(D)-Ala);
cyclo(Val-Tyr-Xaa-Val-Tyr-(D)-Ala) or
cyclo(Tyr-Val-Xaa-Tyr-Val-(D)-Ala);
 where Xaa is the residue of a natural amino acid other than cysteine and tryptophan,
and the physiologically tolerated salts thereof.

4. A compound of the formula 
cyclo(Tyr-Val-Ala-Gly-Xaa-(D)-Ala);
cyclo(Tyr-Val-Ala-Gln-Xaa-(D)-Ala);
cyclo(Tyr-Val-Ala-Lys-Xaa-(D)-Ala);
cyclo(Tyr-Val-Ala-Asn-Xaa-(D)-Ala);
cyclo(Tyr-Val-Ala-Ile-Xaa-(D)-Ala);
cyclo(Tyr-Val-Ala-Leu-Xaa-(D)-Ala);
cyclo(Tyr-Val-Ala-Pro-Xaa-(D)-Ala);
cyclo(Tyr-Val-Ala-Phe-Xaa-(D)-Ala);
cyclo(Tyr-Val-Ala-His-Xaa-(D)-Ala);
cyclo(Tyr-Val-Ala-Ala-Xaa-(D)-Ala) or
cyclo(Tyr-Val-Ala-Tyr-Xaa-(D)-Ala);
 where Xaa is the residue of a natural amino acid other than cysteine and tryptophan,
and the physiologically tolerated salts thereof.

5. A compound of the formula 
cyclo(Val-Tyr-Ala-Val-Tyr-(D)-Ala);
cyclo(Val-Tyr-Arg-Val-Tyr-(D)-Ala);
cyclo(Val-Tyr-Asn-Val-Tyr-(D)-Ala);
cyclo(Val-Tyr-Asp-Val-Tyr-(D)-Ala);
cyclo(Val-Tyr-Gln-Val-Tyr-(D)-Ala);
cyclo(Val-Tyr-Glu-Val-Tyr-(D)-Ala);
cyclo(Val-Tyr-Gly-Val-Tyr-(D)-Ala);
cyclo(Val-Tyr-His-Val-Tyr-(D)-Ala);
cyclo(Val-Tyr-Ile-Val-Tyr-(D)-Ala);
cyclo(Val-Tyr-Leu-Val-Tyr-(D)-Ala);
cyclo(Val-Tyr-Lys-Val-Tyr-(D)-Ala);
cyclo(Val-Tyr-Met-Val-Tyr-(D)-Ala);
cyclo(Val-Tyr-Phe-Val-Tyr-(D)-Ala);
cyclo(Val-Tyr-Pro-Val-Tyr-(D)-Ala);
cyclo(Val-Tyr-Ser-Val-Tyr-(D)-Ala);
cyclo(Val-Tyr-Thr-Val-Tyr-(D)-Ala);
cyclo(Val-Tyr-Tyr-Val-Tyr-(D)-Ala) or
cyclo(Val-Tyr-Val-Val-Tyr-(D)-Ala);
 and the physiologically tolerated salts thereof.

6. A compound of the formula 
cyclo(Val-Tyr-Ala-Val-Tyr-(D)-Ala);
cyclo(Tyr-Val-Ala-Tyr-Val-(D)-Ala);
 
cyclo(Tyr-Val-Hyp-Tyr-Val-(D)-Ala);
cyclo(Tyr-Val-Gln-Tyr-Val-(D)-Ala);
cyclo(Tyr-Val-Lys-Tyr-Val-(D)-Ala);
cyclo(Tyr-Val-Glu-Tyr-Val-(D)-Ala);
cyclo(Tyr-Val-Asp-Tyr-Val-(D)-Ala);
cyclo(Tyr-Val-Phe-Tyr-Val-(D)-Ala);
cyclo(Tyr-Val-Gly-Tyr-Val-(D)-Ala);
cyclo(Tyr-Val-His-Tyr-Val-(D)-Ala);
cyclo(Tyr-Val-Ile-Tyr-Val-(D)-Ala);
cyclo(Tyr-Val-Leu-Tyr-Val-(D)-Ala);
cyclo(Tyr-Val-Asn-Tyr-Val-(D)-Ala);
cyclo(Tyr-Val-Pro-Tyr-Val-(D)-Ala);
cyclo(Tyr-Val-Arg-Tyr-Val-(D)-Ala);
cyclo(Tyr-Val-Ser-Tyr-Val-(D)-Ala);
cyclo(Tyr-Val-Thr-Tyr-Val-(D)-Ala);
cyclo(Tyr-Val-Val-Tyr-Val-(D)-Ala);
cyclo(Tyr-Val-Tyr-Tyr-Val-(D)-Ala) or
cyclo(Tyr-Val-Met-Tyr-Val-(D)-Ala);
 and the physiologically tolerated salts thereof.

7. A process for preparing a compound of the formula I as claimed in one or more of claims 1-6, which comprises
a) coupling suitably protected amino acid derivatives to a solid support,
b) cleaving the linear peptide which has been formed from the support while retaining the side-chain protective groups,
c) cyclizing the linear peptide in solution,
d) eliminating the side-chain protective groups, and, where appropriate,
e) converting it into a physiologically tolerated salt.

8. A mixture comprising compounds as claimed in one or more of claims 1-6.

9. A process for preparing a mixture as claimed in claim 8, which comprises
a) coupling suitably protected amino acid derivatives to a solid support, with, for the purpose of introducing variable positions, either addition of a mixture of all the desired amino acids or division of the support, together with the oligopeptides coupled to it, into fractions and coupling of each fraction individually to a specific amino acid and then pooling of the fractions once again,
b) cleaving the mixture of linear peptides which has been formed from the support while retaining the side-chain protective groups,
c) cyclizing the mixture of linear peptides in solution, and
d) eliminating the side-chain protective groups and, where appropriate,
e) converting it into a physiologically tolerated salt.

10. The use of a compound of the formula I as claimed in one or more of claims 1-6 for preparing a medicament.

11. The use of a compound of the formula I as claimed in one or more of claims 1-6 for preparing a medicament for the treatment or prophylaxis of allergies and infections.

12. The use of a compound of the formula I as claimed in one or more of claims 1-6 for preparing a diagnostic agent.

13. The use of a compound of the formula I as claimed in one or more of claims 1-6 for preparing a scientific tool for inhibiting the binding of interleukin-4 (IL-4) to the IL-4 receptor.

14. A pharmaceutical or diagnostic agent comprising an effective quantity of a compound of the formula I as claimed in one or more of claims 1-6.

15. A process for preparing a pharmaceutical or diagnostic agent as claimed in claim 14, which comprises mixing at least one compound as claimed in one or more of claims 1-6 with a physiologically acceptable excipient and, where appropriate, suitable additives and/or auxiliary substances.

16. The use of a mixture as claimed in claim 8 for preparing a medicament for the treatment or prophylaxis of allergies and infections.

17. The use of a mixture as claimed in claim 8 for preparing a diagnostic agent.

18. The use of a mixture as claimed in claim 8 for preparing a scientific tool for inhibiting the binding of interleukin-4 (IL-4) to the IL-4 receptor.

19. A pharmaceutical or diagnostic agent comprising an effective quantity of a mixture as claimed in claim 8.

20. A process for preparing a pharmaceutical or diagnostic agent as claimed in claim 19, which comprises mixing one or more mixtures as claimed in claim 8 with a physiologically acceptable excipient and, where appropriate, suitable additives and/or auxiliary substances.

## Revendications

1. Composé de formule I cyclo(A-B-C-E-F-(D)-Ala)     (I) dans laquelle A et B peuvent être identiques ou différents et représentent Ile, Leu, Val, Phe ou Tyr, et C, E et F peuvent être identiques ou différents et représentent le reste d'un aminoacide naturel hormis la cystéine et le tryptophane,
et sels physiologiquement acceptables d'un tel composé.

2. Composé de formule I selon la revendication 1, dans lequel A et B peuvent être identiques ou différents et représentent Ile, Leu, Val, Phe ou Tyr, et C, E et F peuvent être identiques ou différents et représentent Ala, Arg, Asp, Asn, Gln, Glu, Gly, His, Ile, Leu, Lys, Met, Phe, Pro, Ser, Thr, Tyr ou Val,
et sels physiologiquement acceptables d'un tel composé.

3. Composé de formule 
cyclo(Val-Val-Xaa-Val-Val-(D)-Ala),
cyclo(Val-Tyr-Xaa-Val-Tyr-(D)-Ala) ou
cyclo(Tyr-Val-Xaa-Tyr-Val-(D)-Ala),
 Xaa représentant le reste d'un aminoacide naturel hormis la cystéine et le tryptophane,
et sels physiologiquement acceptables d'un tel composé.

4. Composé de formule 
cyclo(Tyr-Val-Ala-Gly-Xaa-(D)-Ala),
cyclo(Tyr-Val-Ala-Gln-Xaa-(D)-Ala),
cyclo(Tyr-Val-Ala-Lys-Xaa-(D)-Ala),
cyclo(Tyr-Val-Ala-Asn-Xaa-(D)-Ala),
cyclo(Tyr-Val-Ala-Ile-Xaa-(D)-Ala),
cyclo(Tyr-Val-Ala-Leu-Xaa-(D)-Ala),
cyclo(Tyr-Val-Ala-Pro-Xaa-(D)-Ala),
cyclo(Tyr-Val-Ala-Phe-Xaa-(D)-Ala),
cyclo(Tyr-Val-Ala-His-Xaa-(D)-Ala),
cyclo(Tyr-Val-Ala-Ala-Xaa-(D)-Ala) ou
cyclo(Tyr-Val-Ala-Tyr-Xaa-(D)-Ala),
 Xaa représentant le reste d'un aminoacide naturel hormis la cystéine et le tryptophane,
et sels physiologiquement acceptables d'un tel composé.

5. Composé de formule 
cyclo(Val-Tyr-Ala-Val-Tyr-(D)-Ala),
cyclo(Val-Tyr-Arg-Val-Tyr-(D)-Ala),
cyclo(Val-Tyr-Asn-Val-Tyr-(D)-Ala),
cyclo(Val-Tyr-Asp-Val-Tyr-(D)-Ala),
cyclo(Val-Tyr-Gln-Val-Tyr-(D)-Ala),
cyclo(Val-Tyr-Glu-Val-Tyr-(D)-Ala),
cyclo(Val-Tyr-Gly-Val-Tyr-(D)-Ala),
cyclo(Val-Tyr-His-Val-Tyr-(D)-Ala),
cyclo(Val-Tyr-Ile-Val-Tyr-(D)-Ala),
cyclo(Val-Tyr-Leu-Val-Tyr-(D)-Ala),
cyclo(Val-Tyr-Lys-Val-Tyr-(D)-Ala),
cyclo(Val-Tyr-Met-Val-Tyr-(D)-Ala),
cyclo(Val-Tyr-Phe-Val-Tyr-(D)-Ala),
cyclo(Val-Tyr-Pro-Val-Tyr-(D)-Ala),
cyclo(Val-Tyr-Ser-Val-Tyr-(D)-Ala),
cyclo(Val-Tyr-Thr-Val-Tyr-(D)-Ala),
cyclo(Val-Tyr-Tyr-Val-Tyr-(D)-Ala) ou
cyclo(Val-Tyr-Val-Val-Tyr-(D)-Ala),
 et sels physiologiquement acceptables d'un tel composé.

6. Composé de formule 
cyclo(Val-Tyr-Ala-Val-Tyr-(D)-Ala),
cyclo(Tyr-Val-Ala-Tyr-Val-(D)-Ala),
cyclo(Tyr-Val-Hyp-Tyr-Val-(D)-Ala),
cyclo(Tyr-Val-Gln-Tyr-Val-(D)-Ala),
cyclo(Tyr-Val-Lys-Tyr-Val-(D)-Ala),
cyclo(Tyr-Val-Glu-Tyr-Val-(D)-Ala),
cyclo(Tyr-Val-Asp-Tyr-Val-(D)-Ala),
cyclo(Tyr-Val-Phe-Tyr-Val-(D)-Ala),
cyclo(Tyr-Val-Gly-Tyr-Val-(D)-Ala),
cyclo(Tyr-Val-His[?]Tyr-Val-(D)-Ala)[?]
cyclo(Tyr-Val-Ile-Tyr-Val-(D)-Ala),
 
cyclo(Tyr-Val-Leu-Tyr-Val-(D)-Ala),
cyclo(Tyr-Val-Asn-Tyr-Val-(D)-Ala),
cyclo(Tyr-Val-Pro-Tyr-Val-(D)-Ala),
cyclo(Tyr-Val-Arg-Tyr-Val-(D)-Ala),
cyclo(Tyr-Val-Ser-Tyr-Val-(D)-Ala),
cyclo(Tyr-Val-Thr-Tyr-Val-(D)-Ala),
cyclo(Tyr-Val-Val-Tyr-Val-(D)-Ala),
cyclo(Tyr-Val-Tyr-Tyr-Val-(D)-Ala) ou
cyclo(Tyr-Val-Met-Tyr-Val-(D)-Ala),
 et sels physiologiquement acceptables d'un tel composé.

7. Procédé pour la préparation d'un composé de formule I selon une ou plusieurs des revendications 1 à 6, caractérisé en ce que
a) des dérivés d'aminoacides convenablement protégés sont couplés à un support solide,
b) on sépare du support le peptide linéaire formé, avec maintien des groupes protecteurs de chaîne latérale,
c) le peptide linéaire est cyclisé en solution,
d) on élimine les groupes protecteurs de chaîne latérale, et éventuellement
e) on le convertit en un sel physiologiquement acceptable.

8. Mélange, contenant des composés selon une ou plusieurs des revendications 1 à 6.

9. Procédé pour la préparation d'un mélange selon la revendication 8, caractérisé en ce que
a) des dérivés d'aminoacides convenablement protégés sont couplés à un support solide, couplage dans lequel, pour l'introduction de positions variables, soit on ajoute un mélange de tous les aminoacides désirés, soit on divise en fractions le support avec les oligopeptides couplés à celui-ci et on assemble chaque fraction individuellement avec un aminoacide déterminé et on réunit ensuite à nouveau les fractions,
b) on sépare du support le mélange formé de peptides linéaires, avec maintien des groupes protecteurs de chaîne latérale,
c) le mélange de peptides linéaires est cyclisé en solution, et
d) on élimine les groupes protecteurs de chaîne latérale, et éventuellement
e) on le convertit en un sel physiologiquement acceptable.

10. Utilisation d'un composé de formule I selon une ou plusieurs des revendications 1 à 6, pour la fabrication d'un médicament.

11. Utilisation d'un composé de formule I selon une ou plusieurs des revendications 1 à 6, pour la fabrication d'un médicament destiné au traitement ou à la prophylaxie d'allergies et d'infections.

12. Utilisation d'un composé de formule I selon une ou plusieurs des revendications 1 à 6, pour la fabrication d'un agent de diagnostic.

13. Utilisation d'un composé de formule I selon une ou plusieurs des revendications 1 à 6, pour la fabrication d'un outil scientifique pour l'inhibition de la liaison de l'interleukine-4 (IL-4) au récepteur d'IL-4.

14. Médicament ou agent de diagnostic, contenant une quantité efficace d'un composé de formule I selon une ou plusieurs des revendications 1 à 6.

15. Procédé pour la fabrication d'un médicament ou d'un agent de diagnostic selon la revendication 14, caractérisé en ce que l'on mélange au moins un composé selon une ou plusieurs des revendications 1 à 6 avec un véhicule physiologiquement acceptable ainsi qu'éventuellement des additifs et/ou adjuvants appropriés.

16. Utilisation d'un mélange selon la revendication 8, pour la fabrication d'un médicament destiné au traitement ou à la prophylaxie d'allergies et d'infections.

17. Utilisation d'un mélange selon la revend.cation 8, pour la fabrication d'un agent de diagnostic.

18. Utilisation d'un mélange selon la revendication 8, pour la fabrication d'un outil scientifique destiné à l'inhibition de la liaison de l'interleukine-4 (IL-4) au récepteur d'IL-4.

19. Médicament ou agent de diagnostic, contenant une quantité efficace d'un mélange selon la revendication 8.

20. Procédé pour la fabrication d'un médicament ou d'un agent de diagnostic selon la revendication 19, caractérisé en ce que l'on mélange un ou plusieurs mélanges selon la revendication 8 avec un véhicule physiologiquement acceptable ainsi qu'éventuellement des additifs et/ou adjuvants appropriés.
